# EUROPÄISCHE PATENTSCHRIFT

(11) **EP 0 499 028 B1**
(45) Veröffentlichungstag und Bekanntmachung des Hinweises auf die Patenterteilung: **29.03.1995**
(21) Anmeldenummer: 92100411.5
(22) Anmeldetag: 13.01.1992
(51) Int. Cl.: A61B 17/34, A61B 1/00

(54) **Trokarhülse mit Ventil**
Trocar sleeve with valve
Manchon de trocart avec clapet

(30) Priorität: 12.02.1991 DE 4104193
(43) Veröffentlichungstag der Anmeldung: 19.08.1992
(73) Patentinhaber: Richard Wolf GmbH, 75438 Knittlingen (DE)
(72) Erfinder: Hiltebrandt, Siegfried, W-7134 Knittlingen (DE)
(74) Vertreter: Vollmann, Heiko, Dipl.-Ing.

(56) Entgegenhaltungen:
- EP-A- 0 323 018
- WO-A-89/09072
- DE-A- 4 002 235

## Beschreibung

Die Erfindung geht von einer Trokarhülse mit einem durch Druck auf die Betätigungshandhabe gegen eine Federung in die Offenlage bringbaren Ventilkolben (Trompetenventil) aus, welcher eine dem Durchmesser des Durchlaßkanals der Trokarhülse entsprechende Durchbohrung aufweist. Bei solchen allgemein bekannten, mit einem Trompetenventil (handbetätigtes Kolbenventil) ausgerüsteten Trokarhülsen verschließt das unter permanenter Federspannung stehende Trompetenventil beim Entfernen des Trokars und beim Auswechseln von Instrumenten den Durchlaß der Trokarhülse gasdicht (vgl. z. B. DE-A-4 002 235).

Bekannte Trokarhülsen dieser Art weisen jedoch den Nachteil auf, daß das unter permanenter Federspannung stehende Trompetenventil in seinem Durchlaß auf das eingeführte Instrument drückt und somit dessen Drehbarkeit und dessen Längsverschiebbarkeit hemmt. Hierdurch wird die feinfühlige Handhabung des Instruments bei der Endoskopie zum Teil erheblich behindert. Außerdem können hierdurch Beschädigungen der Instrumentenoberfläche, besonders bei Instrumenten mit isolierten Oberflächen, auftreten.

Die Aufgabe der Erfindung besteht daher darin, eine Trokarhülse mit Trompetenventil so auszubilden, daß der abgefederte Ventilkolben bei durch die Trokarhülse geführtem Instrument dessen Beweglichkeit nicht hemmt und dessen Oberfläche nicht beschädigt und daß bei einem Wechsel von Instrumenten der Durchlaßkanal der Trokarhülse durch einfache Bedienung schnell gasdicht verschließbar ist.

Diese Aufgabe wird durch die Merkmale des Anspruches 1 gelöst.

Eine spezielle Lösung ist durch die Merkmale der Ansprüche 2 und 3 gekennzeichnet.

Die Erfindung ist nachstehend anhand der Zeichnung erläutert. Es zeigen:
- Figur 1: eine Seitenansicht einer Trokarhülse mit Trompetenventil in der Schließstellung,
- Figur 2: die gleiche Ansicht mit Trompetenventil in der Offenstellung,
- Figur 3: einen vergrößerten Schnitt durch das Trompetenventil nach Figur 1,
- Figur 4: einen vergrößerten Schnitt durch das Trompetenventil nach der Figur 2,
- Figur 5: einen Schnitt durch das Trompetenventil in einer weiteren Ausführungsform in Schließstellung,
- Figur 6: einen Schnitt durch das Trompetenventil nach Figur 5 in Offenstellung.

Nach dem Zeichnungsbeispiel ist im Durchlaßkanal 1 einer Trokarhülse 2 ein bekanntes Trompetenventil 3 angeordnet, welches aus einem Gehäuse 4, 4a mit in ihm verschiebbarem Kolben 5 besteht, der durch eine mit Handhabe 6 versehene Stange 7 mittels einer Feder 8 in die Verschlußlage gedrückt wird, so daß der die Feder 8 aufnehmende zylindrische Teil 5a des Kolbens den Trokarhülsenkanal dichtend absperrt. Der Kolben 5 ist weiter mit einer dem Durchmesser des Durchlaßkanals 1 der Trokarhülse 2 entsprechenden Durchgangsbohrung 5b versehen.

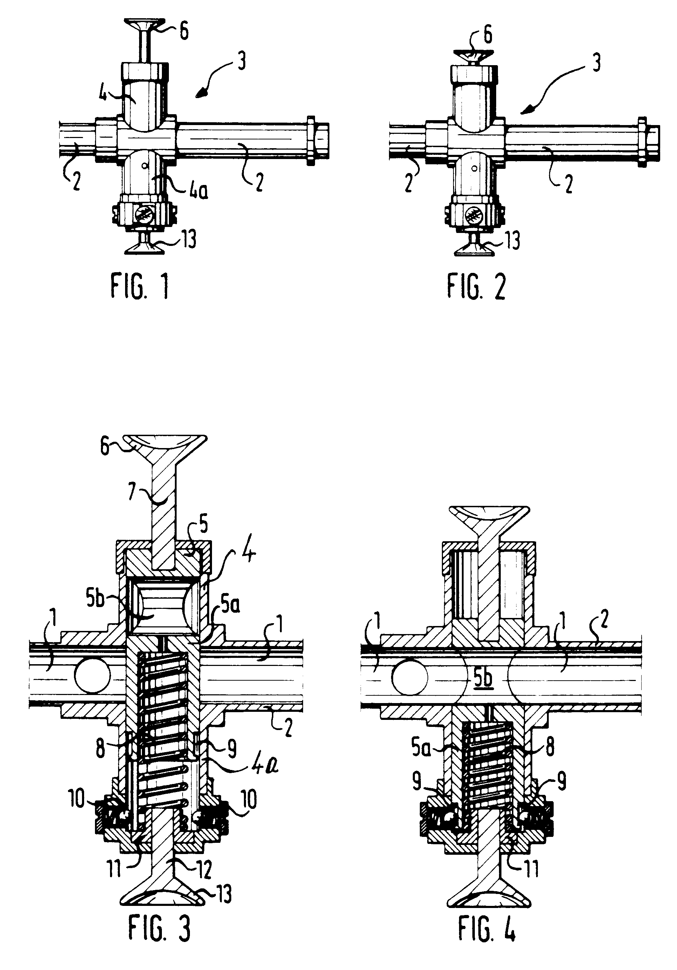

Gemäß der Erfindung ist der zylindrische Kolbenteil 5a in Nähe eines freien Außenendes mit einer oder mehreren Umfangsausnehmungen bzw. mit einer Umfangsnut 9 versehen, und das Ventilgehäuse 4 ist am unteren Ende mit Radialbohrungen zur Aufnahme eines oder mehrerer, radial zur Kolbenachse abgefederter Sperrkugeln 10 versehen, die mit einem Umfangsteil in die Umfangsnut des Kolbens 5, 5a zur Fixierung dessen Lage eingreifen, so daß der Kolben durch Druck auf die Handhabe 6 in die Offenlage nach Figur 4 gedrückt ist, in der die Bohrung 5b des Kolbens 5 mit dem Durchgangskanal 1 der Trokarhülse 2 fluchtet. In dieser fixierten Offenlage können Instrumente durch die Trokarhülse in eine Körperhöhle eingeführt werden ohne daß die Instrumente in ihrer Beweglichkeit innerhalb der Trokarhülse gehemmt oder beschädigt werden.

Um beim Wechsel von Instrumenten den Kanal 1 einfach und schnell verschliessen zu können, wird im Gehäuseteil 4a eine Scheibe 11 oder ein Kolben gelagert, der mit einer durch den Verschluß des Gehäuseteiles 4a greifenden Stange 12 mit Handhabe 13 verbunden ist. Durch Druck auf die Handhabe 13 verschiebt die Scheibe 11 den Kolben 5, 5a gegen die Wirkung der dann aus der Nut 9 zurückgedrückten Sperrkugeln 10, so daß der Kolben frei wird und durch seine Abfederung 8 in die Schließlage entsprechend Figur 3 zurückgedrückt wird, wodurch der Durchlaßkanal 1 der Trokarhülse 2 gasdicht gegen die Körperhöhle abgeschlossen wird.

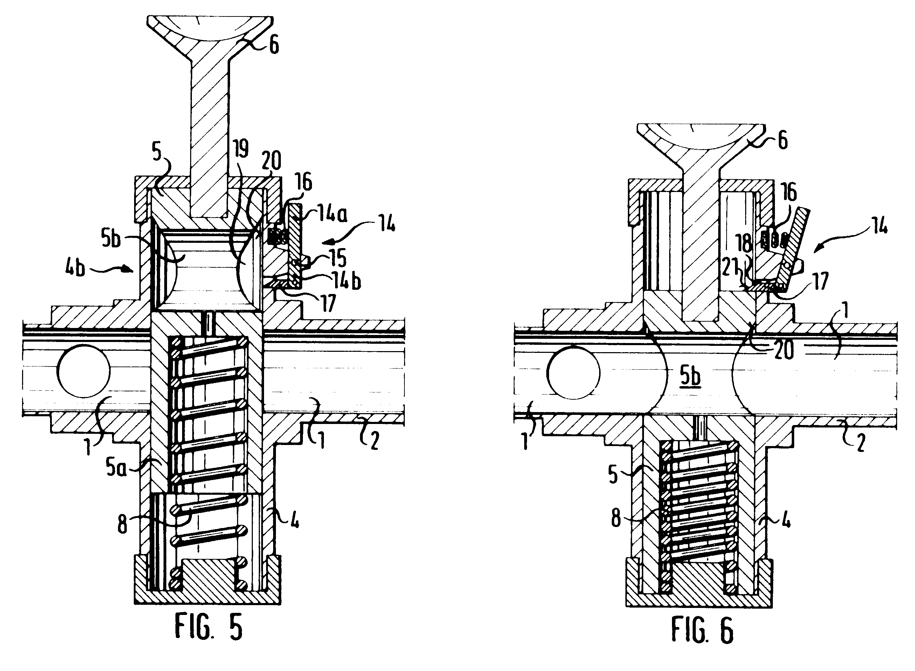

Nach einer weiteren Ausführungsform der Erfindung wird die Sperre zur Fixierung des Ventilkolbens 5, 5a im oberen Ventilgehäuseteil 4b vorgesehen. Erfindungsgemäß ist, wie in den Figuren 5 und 6 dargestellt, am oberen Ventilgehäuseteil 4b ein doppelarmiger Hebel 14 um einen Zapfen 15 schwenkbar angebracht, wobei der eine Hebelarm 14a mittels einer Feder 16 gegen das Ventilgehäuse abgestützt ist und der andere Hebelarm 14b mit einem Zapfen 17 über ein Gelenk verbunden ist. Der Zapfen 17 ragt durch eine Bohrung 18 in das Ventilgehäuse hinein und bildet dadurch, wie in Figur 6 dargestellt, die Sperre für den in die Offenlage gedrückten Kolben 5. In dieser Stellung fluchtet wieder die Bohrung 5b des Kolbens 5 mit dem Durchgangskanal 1 der Trokarhülse und in die Trokarhülse eingeführte Instrumente bleiben in ihrer Beweglichkeit innerhalb der Trokarhülse ungehemmt und werden nicht beschädigt.

Zum schnellen und einfachen Verschließen des Durchgangskanals der Trokarhülse, z. B. beim Instrumentenwechsel, wird der Hebel 14 gegen den Druck der Feder 16 betätigt, wodurch der Zapfen 17 soweit aus dem Inneren des Ventilgehäuses 4 in die Bohrung 18 zurückgezogen wird, daß der Ventilkolben 5 unter der Einwirkung der Rückstellkraft der Feder 8 in die in Figur 5 dargestellte Schließstellung gleiten kann. Der Durchgangskanal 1 der Trokarhülse ist somit wieder gasdicht abgeschlossen. Nach dem Loslassen des Hebels 14 schiebt sich unter der Rückstellkraft der Feder 16 der Zapfen 17 durch die Bohrung 18 in den sich im Bereich der Kolbenbohrung 5b befindenden Freiraum 19 zwischen Ventilgehäuse 4 und Kolben 5 vor.

Zum erneuten Übergang in die Offenstellung der erfindungsgemäßen Trokarhülse wird durch Betätigen des Hebels 14 der Zapfen 17 aus dem Freiraum 19 in die Bohrung 18 zurückgezogen und durch Druck auf die Handhabe 6 wird dann der Kolben 5 in die Offenstellung gebracht und in dieser Stellung nach Loslassen des Hebels 14, wie oben beschrieben, in der Offenstellung fixiert ist.

Die Offenstellung kann auch lediglich durch Betätigen des Kolbens 5 über die Handhabe 6 erreicht werden, wenn nach einer vorteilhaften Ausgestaltung dieser zweiten Ausführungsform der Kolben 5 und der Zapfen 17 mit korrespondierenden Auflaufflächen 20 und 21 versehen sind. Beim Betätigen des Kolbens aus der Schließstellung gleiten diese korrespondierenden Auflaufflächen 20,21 aneinander vorbei und der Zapfen 17 wird in die Bohrung 18 zurückgedrückt und springt aufgrund der Rückstellkraft der Feder 16, nachdem der Kolben 5 die Bohrung 18 passiert bat, in die in Figur 6 dargestellte Position, wodurch der Kolben 5 wieder in der Offenstellung fixiert ist.

## Patentansprüche

1. Trokarhülse mit einem durch Druck auf eine Betätigungshandhabe gegen eine Federung in die Offenlage bringbaren Kolben eines Kolbenventils, welcher eine dem Durchmesser des Durchlaßkanals der Trokarhülse entsprechenden Durchbohrung aufweist, dadurch gekennzeichnet, daß der Ventilkolben (5, 5a) gegen seine Abfederung (8) durch eine von Hand lösbare Sperre in seiner Offenlage im Ventilgehäuse (4, 4a) fixierbar ist,

2. Trokarhülse nach Anspruch 1, dadurch gekennzeichnet, daß der zylindrische, die Kolbenabfederung (8) aufnehmende Kolbenteil (5a) in Nähe seines freien Außenendes mit mindestens einer Ausnehmung (9) versehen ist, in die je eine in der Ventilgehäusewand (4a) radial abgefedert beweglich gelagerte Kugel (10) mit einem Umfangsteil eingreift, und daß der Ventilkolben durch einen von Hand in Richtung der Kolbenfederung (8) betätigbaren Mitnehmer (11) zur Aufhebung der Kugelsperre verschiebbar ist.

3. Trokarhülse nach Anspruch 1 oder 2, dadurch gekennzeichnet, daß die Ausnehmung (9) in Nähe des Außenendes des zylindrischen Kolbenteiles (5a) aus einer umlaufenden Nut (9) besteht, in die mindestens eine in der Ventilgehäusewand (4a) radial abgefedert gelagerte Sperrkugel (10) mit einem Umfangsteil eingreifen kann.

4. Trokarhülse nach Anspruch 1, dadurch gekennzeichnet, daß die Sperre aus einem federvorgespannten doppelarmigen Hebel (14), der an dem Ventilgehäuse (4) auf der der Ventilhandhabe (6) zugekehrten Seite der Trokarhülse (2) angelenkt ist, und aus einem Sperrzapfen (17) besteht, der an dem Hebel (14) angelenkt ist und in Offenstellung des Ventils (3) mit dessen Kolben (5) sperrend zusammenwirkt.

5. Trokarhülse nach Anspruch 4, dadurch gekennzeichnet, daß der Sperrzapfen (17) und der Kolben (5) je mit einer Auflauffläche (21 bzw. 20) versehen sind.

## Claims

1. A trocar sleeve with a plunger of a plunger valve, which plunger, by pressure onto an actuating handle against a spring, is able to be brought into the open position, which plunger has a through bore corresponding to the diameter of the passage channel of the trocar sleeve, characterised in that the valve plunger (5,5a) is able to be fixed in its open position in the valve housing (4,4a) against its springing (8) by a closure which is able to be released by hand.

2. A trocar sleeve according to Claim 1, characterised in that the cylindrical plunger part (5a) receiving the plunger springing (8) is provided in the vicinity of its free outer end with at least one recess (9), into which in each case a ball (10) engages with part of its circumference, which ball is mounted so as to be movable in a radially sprung manner in the valve housing wall (4a), and that the valve plunger is able to be displaced by a carrier (11) which is able to be actuated by hand in the direction of the plunger springing (8) to lift the ball closure.

3. A trocar sleeve according to Claim 1 or 2, characterised in that the recess (9) in the vicinity of the outer end of the cylindrical plunger part (5a) consists of an encircling groove (9), into which at least one closure ball (10), mounted in a radially sprung manner in the valve housing wall (4a), can engage with a part of its circumference.

4. A trocar sleeve according to Claim 1, characterised in that the closure consists of a spring-biased, two-armed lever (14) which is articulated on the valve housing (4) on the side of the trocar sleeve (2) facing the valve handle (6), and of a blocking pin (17), which is articulated on the lever (14) and in the open position of the valve (3) cooperates with its plunger (5) in a blocking manner.

5. A trocar sleeve according to Claim 4, characterised in that the blocking pin (17) and the plunger (5) are each provided with an abutting surface (21 or 20).

## Revendications

1. Douille de trocart comportant un piston d'une soupape à piston, qui peut être amené dans la position ouverte à l'encontre de l'action d'un ressort, moyennant l'application d'une pression sur une manette d'actionnement et qui comporte un perçage traversant qui correspond au diamètre du canal de passage de la douille de trocart, caractérisée en ce que le piston de soupape (5,5a) peut être fixé dans sa position ouverte dans le boîtier de soupape (4,4a), à l'encontre de son ressort de sollicitation (8), par un dispositif de blocage pouvant être desserré manuellement.

2. Douille de trocart selon la revendication 1, caractérisée en ce que la partie cylindrique (5a) du piston, qui loge le ressort (8) du piston, comporte à proximité de son extrémité extérieure libre au moins un évidement (9), dans lequel une bille (10), qui est montée de manière à être déplaçable radialement dans la paroi (4a) du boîtier de soupape, en étant sollicitée par un ressort, engrène respectivement avec une partie circonférentielle, et en ce que le piston de soupape est déplaçable par un organe d'entraînement (11), qui peut être actionné manuellement en direction du ressort (8) de sollicitation du piston pour supprimer le blocage de la bille.

3. Douille de trocart selon la revendication 1 ou 2, caractérisée en ce que l'évidement (9) situé à proximité de l'extrémité extérieure de la partie cylindrique (5a) du piston est constituée par une gorge circonférentielle (9), dans laquelle au moins une bille de blocage (10), montée de manière à être sollicitée radialement par un ressort dans la paroi (4a) du boîtier de soupape, peut s'engager par une partie circonférentielle.

4. Douille de trocart selon la revendication 1 , caractérisée en ce que le dispositif de blocage est constitué par un levier à deux bras (14), précontraint par un ressort et qui est articulé sur le boîtier (4) de la soupape, sur le côté de la douille de trocart (2) tourné vers la manette (6) d'actionnement de la soupape, et par un téton de blocage (17), qui est articulé sur le levier (14) et, lorsque la soupape (3) est dans sa position ouverte, coopère en réalisant un blocage avec le piston (5) de cette soupape.

5. Douille de trocart selon la revendication 4, caractérisée en ce que le téton de blocage (17) et le piston (5) sont équipés chacun d'une surface en rampe (21 ou 20).
